# EUROPÄISCHE PATENTSCHRIFT

(11) **EP 2 163 143 B1**
(45) Veröffentlichungstag und Bekanntmachung des Hinweises auf die Patenterteilung: **04.03.2015**
(21) Anmeldenummer: 08784540.0
(22) Anmeldetag: 24.06.2008
(51) Int. Cl.: H05H 1/24

(54) **VORRICHTUNG ZUR BEHANDLUNG VON OBERFLÄCHEN MIT EINEM MITTELS EINER ELEKTRODE ÜBER EIN FESTSTOFF-DIELEKTRIKUM DURCH EINE DIELEKTRISCH BEHINDERTE GASENTLADUNG ERZEUGTEN PLASMA**
DEVICE FOR THE TREATMENT OF SURFACES WITH A PLASMA GENERATED BY AN ELECTRODE OVER A SOLID DIELECTRIC VIA A DIELECTRIC BARRIER GAS DISCHARGE
DISPOSITIF POUR LE TRAITEMENT DE SURFACES À L'AIDE D'UN PLASMA GÉNÉRÉ AU MOYEN D'UNE ÉLECTRODE PAR UNE DÉCHARGE DE GAZ À BARRIÈRE DIÉLECTRIQUE PAR LE BIAIS D'UN DIÉLECTRIQUE SOLIDE

(30) Priorität: 03.07.2007 DE 102007030915
(43) Veröffentlichungstag der Anmeldung: 17.03.2010
(73) Patentinhaber: Cinogy GmbH, 37115 Duderstadt (DE)
(72) Erfinder: WANDKE, Dirk, 37308 Heilbad Heiligenstadt (DE); KAEMLING, Andy, 37318 Birkenfelde (DE); KAEMLING, Cindy, 37318 Birkenfelde (DE)
(74) Vertreter: Lins, Edgar
(86) Internationale Anmeldenummer: PCT/EP2008/005087
(87) Internationale Veröffentlichungsnummer: WO 2009/003613

(56) Entgegenhaltungen:
- EP-A- 0 789 505
- EP-A- 1 047 165
- EP-A- 1 800 711
- WO-A-2005/125287
- WO-A-2006/116252
- CN-A- 1 819 737
- US-A- 3 198 932
- US-A- 4 185 213

## Beschreibung

Die Erfindung betrifft eine Vorrichtung zur Behandlung von Oberflächen nach Anspruch 1.

DE 103 24 92 6 B3 offenbart insbesondere eine Vorrichtung zur Behandlung eines lebende Zellen enthaltenden biologischen Materials mit einem durch eine Gasentladung erzeugten Plasma, bei der eine Elektrode mit Abstand zu dem biologischen Material angeordnet ist. Ein Dielektrikum wird mit Abstand zu dem biologischen Material zwischen der Elektrode und dem biologischen Material angeordnet und zum Zünden der durch das Dielektrikum behinderten Gasentladung zwischen einer aktiven Fläche des Dielektrikums und dem biologischen Material eine Wechselhochspannung an die Elektrode angelegt. Als Dielektrikum wird dabei ein Festkörper-Dielektrikum ohne Abstand vor der Elektrode angeordnet. Nachteilig hierbei ist, dass die biologischen Materialien in der Regel unterschiedliche Topologien aufweisen, so dass aufgrund von nicht homogenen Abständen zur eigentlichen Oberfläche eine unregelmäßige Behandlung für das erzeugte Plasma stattfindet.

DE 101 27 035 A1 offenbart ein Verfahren zur Reinigung der Luft vorwiegend in Wohn- und Büroräumen unter Verwendung der stillen elektrischen Gasentladung, wobei eine Ozonbildung nahezu vollkommen unterdrückt wird und ein diesbezügliches Dielektrikum in einem strukturierten anliegenden Drahtgewebe aus Edelstahl angeordnet ist.

DE 43 02 465 C1 offenbart eine Vorrichtung zur Erzeugung einer dielektrisch behinderten Entladung, mit einem gasgefüllten Entladungsraum zwischen zwei zündspannungsbeaufschlagbaren Elektroden, von denen zumindest eine mit einem Dielektrikum vom Entladungsraum getrennt ist, wobei mindestens eine Elektrode ein spannungsangeregtes Plasma in einem Gas ist, dessen Druck niedriger ist als der Druck im Entladungsraum.

US 4,737,885 offenbart einen Plasmagenerator mit einer Anregungselektrode und einer entgegengesetzt angeordneten Elektrode zum Erzeugen eines Plasmas bei hoher Spannung, wobei zwischen den Elektroden ein poröses Blatt angeordnet ist, das mit dielektrischem Material mit metallischem kornartigen Material versehen ist.

EP 254 111 A1 offenbart einen Hochleistungsstrahler, insbesondere für ultraviolettes Licht, mit einem mit Füllgas gefüllten Entladungsraum, dessen Wandungen zum einen durch ein Dielektrikum gebildet sind, welches auf seiner dem Entladungsraum abgewandten Oberfläche mit ersten Elektroden versehen ist, zum anderen aus zweiten Elektroden oder gleichfalls durch ein Dielektrikum gebildet sind, welches auf seiner dem Entladungsraum abgewandten Oberfläche mit zweiten Elektroden versehen ist, mit einer an die ersten und zweiten Elektroden angeschlossenen Wechselstromquelle zur Speisung der Entladung sowie Mitteln zur Leitung der durch die stille elektrische Entladung erzeugten Strahlung in einen Außenraum, wobei sowohl das Dielektrikum als auch die ersten Elektroden für die besagte Strahlung durchlässig sind.

DE 195 32 105 C2 offenbart ein Verfahren zur Behandlung von dreidimensionalen Werkstücken mit einer direkten wechselspannungsinduzierten Barriereentladung, bei dem die zu behandelnde Werkstückoberfläche einer ersten der wechselspannungsinduzierten Barriereentladung dienenden Elektrode gegenüberliegend angeordnet wird, die werk-stückseitig eine elektrisch isolierende Barriere aufweist, bei dem das Werkstück als zweite der wechselspannungsinduzierenden Barriereentladung dienende Elektrode eingesetzt wird und bei dem wenigstens die Barriere mit einer der zu behandelnden Werkstückoberfläche nachgebildeten Oberfläche verwendet wird, wobei die Herstellung der die Oberfläche nachbildenden Barriere hergestellt wird durch Abgießen insbesondere mittels thermoplastischen Kunststoffes. Hierbei muss für jede neue Topologie ein entsprechender Abguss vorab durchgeführt werden, welches mit einem relativ hohen Zeit-, Material- und somit Kostenaufwand verbunden ist.

Durch US 6,455,014 B1 ist eine Vorrichtung zur Behandlung von Oberflächen von Objekten bekannt, bei der eine drahtförmige Elektrode von einem schlauchförmigen Dielektrikum umschlossen ist, auf dem seinerseits eine Gegenelektrode angeordnet wird. Die Anordnung dient dazu, an den Kreuzungsstellen des Dielektrikums der Elektrode und der Gegenelektrode eine CoronaEntladung zu bewirken, um so lokal begrenzte Plasmafelder entstehen zu lassen. Die Anordnung wird so gewählt, dass die Plasmafelder einander überlappen, sodass eine ausgedehnte Plasmaquelle realisiert wird. Insbesondere können dabei zueinander parallel verlaufende, dielektrisch abgeschirmte Elektroden einerseits und zueinander parallel verlaufende unabgeschirmte Gegenelektroden andererseits ein Gitternetz bilden, um so eine Quelle für ein flächiges Plasmafeld darzustellen, in das die zu behandelnde Oberfläche eingebracht werden kann. Die Elektroden ragen dabei an beiden Enden aus dem schlauchförmigen Dielektrikum heraus, um in geeigneter Weise kontaktiert zu werden.

Eine Vorrichtung der eingangs erwähnten Art ist durch EP 0 789 505 A1 bekannt. Die Vorrichtung ist als ein tragbares Gerät ausgebildet, dass eine Schalteinrichtung zur Erzeugung einer Wechselspannung aufweist. Die Wechselspannung wird über einen Tesler-Transformator hochtransformiert, an den ein zu einer Schlaufe geformter Elektrodendraht angeschlossen ist. Der Elektrodendraht ist von einem Teflonschlauch umgeben. Die Enden des Elektrodendrahts und des Teflonschlauches werden in dem Gerät gehalten. Die Vorrichtung dient zur Behandlung von Oberflächen durch eine Corona-Entladung.

Das der Erfindung zugrundeliegende Problem liegt daher darin, eine gattungsgemäße Vorrichtung bereitzustellen, die bei unterschiedlichsten Topologien, insbesondere der Oberflächen, der zu behandelnden Materialien, Insbesondere von Haut, eine gleichmäßige Behandlung über ein erzeugtes Plasma gewährleistet.

Dieses Problem wird erfindungsgemäß durch eine Vorrichtung nach Anspruch 1 gelöst.

Die erfindungsgemäße Vorrichtung ist dadurch gekennzeichnet, dass diese eine flexible Wirk-Oberfläche aufweist, die während der Behandlung unmittelbar an das Plasma angrenzt.

Erfindungsgemäß ist unter dem Begriff Wirk-Oberfläche eine Oberfläche der Vorrichtung zu verstehen, die während der Behandlung - also bei existierendem Plasma - unmittelbar an das Plasma angrenzt und aufgrund der allgemeinen Materialeigenschaften das Material eine Dielektrizitätskonstante von ungleich Null aufweist, so daß eine dielektrische Behinderung der Gasentladung und somit eine entsprechende Wirkung stattfindet. Das Dielektrikum selbst ist vom Aggregatzustand her fest und kann, muß jedoch nicht, mit einem oder mehreren Materialien beschichtet sein, welche erst eine gewisse Flexibilität ermöglicht, beispielsweise, wenn das Dielektrikum zwar fest jedoch als Pulver vorliegt und dieses Pulver aufgebracht bzw. eingebracht ist in ein kautschukähnliches Material, welches viskoelastische Eigenschaften aufweist und entsprechend geformt werden kann.

Erfindungsgemäß ist bezüglich der Wirk-Oberfläche bzw. der Oberfläche des Dielektrikums eine reversible Formveränderbarkeit zu verstehen. Somit ist eine mechanische Anpaßbarkeit an die örtlichen Gegebenheiten möglich bei gleichzeitiger Wirkung hinsichtlich der Behinderung einer dielektrischen Behinderung hinsichtlich einer entsprechenden Gasentladung. Selbstverständlich ist es auch denkbar, daß ein körniges oder pulverförmiges festes Dielektrikum auf einem flexiblen Träger sich befindet/angeordnet ist.

Wie bereits oben erwähnt, ist es jedoch auch denkbar, daß - quasi diametral dazu - ein an sich starres - im Sinne von Nichtbiegsamkeit - vorhandenes Feststoff-Dielektrikum mit einer Beschichtung versehen ist, die als solche flexibel ist bzw. die Flexibilität als solche erst ermöglicht bzw. verbessert, so daß in Bezug auf die Wirkung des Feststoffdielektrikums und seiner Ausgestaltung im erfindungsgemäßen Sinne eine flexible Wirk-Oberfläche bereitgestellt wird.

Das Grundprinzip der erfindungsgemäßen Vorrichtung beruht darauf, ein zu behandelndes Objekt einem mittels einer Elektrode und Gegenelektrode erzeugten Plasmas zu unterwerfen, wobei zwischen dem zu behandelnden Objekt und der Elektrode ein Dielektrikum angeordnet ist, so daß ein Plasma mittels einer dielektrisch behinderten Gasentladung erzeugt wird, wobei dieses dann auf das zu behandelnde Objekt angewandt wird.

Durch dieses Anregungsprinzip entsteht zwischen Elektrode und Behandlungsareal eine kalte Gasentladung (Plasma). Dadurch wird es ermöglicht, Oberflächen und/oder Hohlräume in einem geringen Abstand (0,1 - 50 mm), also berührungslos und/oder anliegend in einem lokal eng begrenzten Bereich und/oder durch Aneinanderreihung mehrerer flexibler Elektroden oder einer gewebeartigen Struktur auch großflächig mit unterschiedlicher Topologie zu behandeln. Aus den speziellen Eigenschaften des Plasmas ergeben sich neben einer Anwendung zur Behandlung, Aktivierung, Funktionalisierung und Desinfektion der entsprechenden Oberflächen und/oder Hohlräume auch Einsatzgebiete im medizinischen Bereich, insbesondere bei der Anwendung auf Haut ober aber auch für innere Anwendungen.

Die dabei nutzbaren Effekte setzen sich beispielsweise aus einer geringen Dosis UV-Bestrahlung im nützlichen UV-A- bzw. UV-B-Wellenlängenbereich und aus den reaktiven Gasspezies in der Gasentladung (Plasma) zusammen. Damit kombiniert das Verfahren mehrere wirkungsvolle Effekte, woraus eine Minderung des Juckreizes, eine Förderung der Mikrozirkulation, eine immunmodulatorische Wirkung und eine bakterizide und fungizide Wirkung resultiert, was wiederum bei einer Applikation für zumindest einen Teil von Schuheinlagen sehr nützlich ist. Gleichzeitig kann die Vorrichtung auch zum Abtasten von Oberflächen und/oder Hohlräumen, insbesondere von Haut, verwendet werden, da dadurch die Behandlung von Hautkrankheiten mit begleitendem, intensiven Juckreiz aber auch die Behandlung von chronischen Wundheilungsstörungen auf der Basis von Mikrozirkulationsstörungen ermöglicht wird.

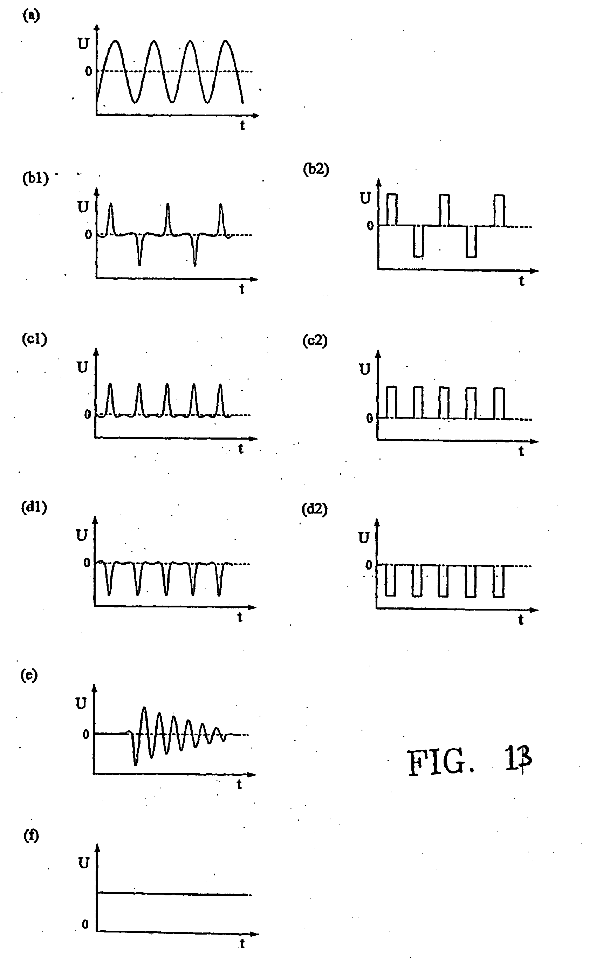

Bei der erfindungsgemäßen Vorrichtung wird mit Spannungen im Bereich von 100 bis 100.000 Volt gearbeitet. Die angelegte Spannung (siehe Figur 13) kann sinusförmig (a), pulsförmig (b1,b2,c1,c2,d1,d2) (unipolar oder bipolar), die Form eines Hochfrequenzpulses (e) oder die Form einer Gleichspannung (f) aufweisen. Auch Kombinationen von unterschiedlichen Spannungsformen sind einsetzbar. Die Elektrode kann aus elektrisch gut leitenden Materialien bestehen, wobei die Gegenelektrode aus den selben Materialien bestehen und/oder das zu behandelnde Objekt bildet die Gegenelektrode. Üblicherweise bestehen die Festkörperdielektrika aus Gläsern, Keramiken oder Kunststoffen.

Die Wechselspannungfrequenz beträgt üblicherweise 1 bis 100.000.000s⁻¹. Die Einwirkzeiten der Plasmabehandlung richten sich nach dem Einsatzgebiet und können von einigen Millisekunden über mehrere Minuten bis hin zu einigen Stunden betragen.

Erfindungswesentlich ist u. a. die Tatsache, daß die Vorrichtung eine flexible Wirk-Oberfläche aufweist, die während der Behandlung unmittelbar an das Plasma angrenzt, insbesondere, wenn das Feststoffdielektrikum mit einer flexiblen Oberfläche ausgestattet ist, was beispielsweise dadurch bewerkstelligt werden kann, daß das Dielektrikum als Granulat und/oder als Pulver ausgestaltet ist. Dies kann jedoch auch dadurch realisiert werden, daß das Dielektrikum, beispielsweise als feines Pulver, an und/oder in einer flexiblen Hohlfaser, beispielsweise aus Gläsern, Keramiken oder Kunststoffen angeordnet ist oder das Dielektrikum selbst eine flexible Hohlfaser darstellt. Die Hohlfaser kann einen Innendurchmesser von 0,5*µ*m bis 2000*µ*m besitzen. Die Wandstärken liegen im Bereich von 10*µ*m bis 2000*µ*m. Die Länge der Hohlfasern und die damit verbundene effektive aktive Länge kann von einigen Millimetern bis zu einigen Metern betragen. Die Sicherstellung der elektrischen Verbindung von einem Anschluß zur Elektrode bzw. Gegenelektrode erfolgt insbesondere und beispielsweise über eine metallische Kontaktierung am Ende der Hohlfaser. Diese ist beispielsweise und insbesondere derart in die Hohlfaser eingebracht, daß sie diese verschließt, wenn notwendig auch gasdicht, und somit eine leitende Verbindung ermöglicht. Hohlfaser, Kontaktierung und Anschluß sind derart in einer Halterung untergebracht, daß eine sichere Verbindung von der Spannungsversorgung zur Kontaktierung ermöglicht wird.

Aufgrund der Flexibilität der Wirk-Oberfläche kann die Vorrichtung auch in schwierigen Situationen wie beispielsweise Hohlräumen - wie bei offenen Wunden - deart appliziert werden, daß eine gleichmäßige und homogene Einwirkung des Plasmas auf die zu behandelnde Oberfläche gewährleistet ist.

In diesem Zusammenhang ist es vorteilhaft, wenn die Elektrode zumindest teilweise direkt an der Oberfläche des Dielektrikums anliegt, um eine möglichst hohe Feldstärke des zwischen Elektrode und Gegenelektrode aufgebauten elektrischen Feldes im Dielektrikum und bei Anordnung der zu behandelnden Oberfläche eines bestimmten Objektes/Subjektes, beispielsweise bei Haut, sich zwischen Elektrode und Gegenelektrode befindend, aufzubauen.

In diesem Zusammenhang und als alternative Ausführungsform ist es vorteilhaft, wenn die Elektrode zumindest teilweise mittels eines Spacers beabstandet zur Oberfläche des Dielektrikums ist. Da auf diese Weise bei Ausgestaltung des Spacers als leitfähiges Material und somit nicht als Dielektrikum, so daß bei entsprechender Auslegung des Spacers hinsichtlich seiner elektrischen Leitfähigkeit zwischen der elektrischen Leitfähigkeit der Elektrode (sehr gut leitend) und der elektrischen Leitfähigkeit des Dielektrikums (schlecht bis isolierend wirkend) liegt, um auf diese Weise die elektrischen Feldvektoren zu homogenisieren, was zu einer besseren und gleichmäßigeren flächenmäßigeren Ausbreitung des Plasmas führt.

In diesem Kontext ist es vorteilhaft, wenn die Elektrode zumindest teilweise als Beschichtung am Dielektrikum anliegt, da auf diese Weise insbesondere bei Ausgestaltung des Dielektrikums als flexibler Hohlleiter eine hochgradig flexible Ausgestaltung realisiert wird.

Denkbar ist jedoch auch, daß die Elektrode vollmaterialig ausgestaltet ist, so daß bei Ausgestaltung des Dielektrikums als flexible Hohlfaser die Elektrode als Vollmaterial sicher in der flexiblen Hohlfaser angeordnet ist.

Weiterhin ist es vorteilhaft, wenn die Elektrode als Granulat und/oder als Pulver ausgestaltet ist, um auf diese Art und Weise die Flexibilität (beispielsweise Biegbarkeit) zumindest eines Teils der Vorrichtung zu gewährleisten.

Denkbar und vorteilhaft ist es jedoch auch, wenn die Elektrode im Betriebszustand ein ionisiertes Gas ist, so daß bei einer entsprechenden Ausgestaltung des Dielektrikums an und/oder in einer flexiblen Hohlfaser bzw. als Hohlfaser selbst eine besonders hohe Flexibilität (u. a. Biegbarkeit) der Faser gegeben ist, da kein Kernmaterial als Feststoff vorliegt; entsprechendes gilt bei einer Ausgestaltung der Elektrode als elektrisch leitendes Fluid, beispielsweise und insbesondere als wäßrige Salzlösung.

Bei der Applikation des Plasmas auf eine Oberfläche ist es besonders vorteilhaft, wenn die erfindungsgemäße Vorrichtung eine Gegenelektrode aufweist, da auf diese Art und Weise die Applikation und Führung des Plasmas besser gesteuert werden kann, im Gegensatz zu Ausführungsformen, bei denen das zu behandelnde Objekt quasi als Gegenelektrode fungiert.

Weiterhin ist es vorteilhaft, wenn die erfindungsgemäße Vorrichtung eine insbesondere flexible Gasabsaugeinrichtung und/oder eine insbesondere flexible Gaszuführungseinrichtung aufweist, um auf diese Weise das erzeugte Plasma mittels Gasentladung gezielt steuern zu können, um beispielsweise eventuell unerwünschte Sauerstoffradikale oder Stickoxide so schnell wie möglich zu entfernen bzw. um gezielt Gase zuzuführen, um beispielsweise das Behandlungsareal zu kühlen und/oder gezielt Reaktionen auf der Oberläche und/oder in den Hohlräumen hervorzurufen und/oder das Plasma zu stabilisieren. Unter dem Begriff "flexibel" ist die Ausrichtbarkeit und/oder die Platzierbarkeit der entsprechenden Einrichtung zu verstehen, um unterschiedlichen topischen Anforderungen zu entsprechen. Dabei kann es sich beispielsweise bei der Gaszuführungs- als auch der Gasabsaugeinrichtung im wesentlichen um biegsame Schläuche handeln.
Die Gasabsaugeinrichtung bzw. die Gaszuführungseinrichtung kann beispielsweise auch aus flexiblen Hohlfasern gebildet werden, da dies besonders vorteilhaft ist, um dadurch die Flexibilität des Gesamtsystems zu erhalten und/oder zu verbessern.

Schließlich ist es von Vorteil, wenn mindestens die eine Hohlfaser mit sich oder mit mindestens einem anderen Stützelement, beispielsweise in Form von einer Faser, ein gewebeartiges Element, beispielsweise in Form eines Vlieses, bilden, da auf diese Art und Weise eine relativ große zu behandelnde Oberfläche dennoch bei unterschiedlicher Topographie gleichmäßig behandelt werden kann. Ein solches gewebeartiges Element, beispielsweise in Form eines Vlieses, kann in Geweben, und/oder Heilvorrichtungen wie Verbände oder Prothesen eingearbeitet werden.

Die Gewebeform kann ihrem Zweck entsprechend gestaltet sein. Mögliche Formen sind beispielsweise und insbesondere rund oder eckig aufgebaut. Die Oberfläche eines solchen gewebeartigen Elementes kann eine aktive Fläche von 10 mm² bis hin zu 1 m² und mehr aufweisen.

Denkbar und vorteilhaft ist es jedoch auch, wenn flexible Elektroden, insbesondere flexible Gaszuführung und/oder insbesondere flexible Gasabsaugung derart angeordnet sind, daß eine freie Plasmaflamme ausgebildet wird. Die flexiblen Elektroden können dabei einseitig oder beidseitig dielektrisch behindert (abgeschirmt) sein. Mit diesem Beispiel, nicht im Bereich der Erfindung, ist es möglich, Oberflächen und/oder Hohlräume mit einem größeren Abstand als die anderen genannten Ausführungsformen (bis einige cm) mit einem Plasma zu beaufschlagen. Diese Ausführungsform arbeitet unabhängig von der Leitfähigkeit der Oberfläche sowie von dessen Oberflächenstruktur.

Die flexiblen Elektroden ermöglichen zudem, daß die Plasmaflamme durch Aktuatoren und/oder einer Positioniereinheit in X- und/oder Y-Richtung (in einem beliebigen kartesischen System gewählt) ablenkbar ist. Dies ist besonders vorteilhaft, da somit die Plasmaflamme über die Oberfläche geführt werden kann.

Im folgenden wird die Erfindung anhand von in den Figuren dargestellten bevorzugten Ausführungsbeispielen weiter erläutert und beschrieben.
- FIG. 1: stellt skizzenartig das Funktionsprinzip einer Ausführungsform aus dem Stand der Technik dar;
- FIG. 2.: stellt skizzenartig das Funktionsprinzip einer weiteren Ausführungsform aus dem Stand der Technik dar;
- FIG. 3: stellt skizzenhaft das Funktonsprinzip einer dritten Ausführungsform aus dem Stand der Technik dar;
- FIG. 4: stellt skizzenhaft im Querschnitt eine erste erfindungsgemäße Ausführungsform dar;
- FIG. 5: stellt im Querschnitt skizzenhaft eine zweite Ausführungsform der erfindungsgemäßen Vorrichtung dar;
- FIG. 6: stellt im Querschnitt und skizzenhaft eine dritte Ausführungsform der erfindungsgemäßen Vorrichtung dar;
- FIG. 7: stellt im Querschnitt und skizzenhaft eine vierte Ausführungsform der erfindungsgemäßen Vorrichtung dar;
- FIG. 8: stellt skizzenartig und im Querschnitt eine fünfte Ausführungsform der erfindungsgemäßen Vorrichtung dar;
- FIG. 9: stellt skizzenhaft und im Querschnitt eine sechste Ausführungsform der erfindungsgemäßen Vorrichtung dar; stellt
- FIG. 10: eine skizzenhaft und im Querschnitt eine siebte Ausführungsform der erfindungsgemäßen Vorrichtung dar;
- FIG. 11: stellt skizzenartig und im Querschnitt eine medizinische Applikation dar;
- FIG. 12: stellt als Funktionsskizze eine übliche Applikation einer erfindungsgemäßen Vorrichtung dar;
- FIG. 13: stellt skizzenartig verschiedene Spannungsformen dar, die an die Elektrode angelegt werden können;
- FIGUREN 14: stellt skizzenhaft und im Querschnitt achte Ausführungsform dar.
- 15-17: stellen skizzenhaft und im Querchnitt weitere Beispiele, nicht im Bereich der Erfindung, dar.

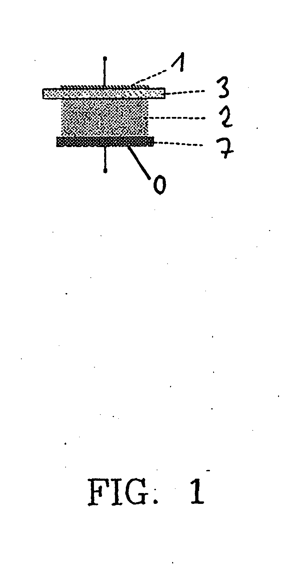

In Figur 1 ist das Funktionsschema der erfindungsgemäßen Vorrichtung - wie aus dem Stand der Technik bekannt - zu erkennen, bei der eine Elektrode (1) und das zu untersuchende Objekt O (leitend) als Gegenelektrode 7 fungierend bei einer Wechselspannung von einigen tausend Volt mit einer Frequenzen bis in den Megehertzbereich ein elektrisches Feld erzeugen, bei dem Luft durch eine entsprechende Gasentladung zu einem Plasma 2 zwischen den Elektroden umgewandelt wird, so daß das zu behandelnde Objekt als Gegenelektrode 7 direkt topisch mit dem Plasma behandelt wird.

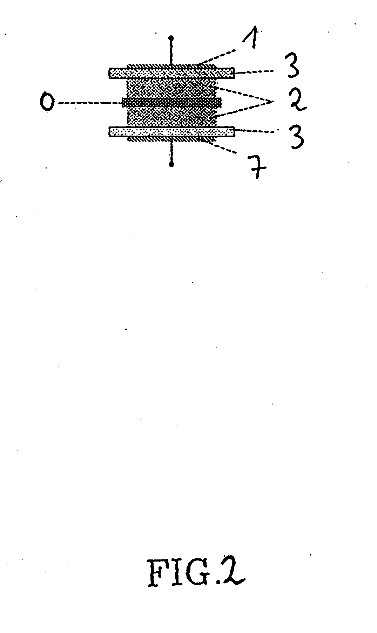

Das in Figur 2 gezeigte Prinzip (Stand der Technik) unterscheidet sich lediglich von dem in Figur 1 offenbarten dahingehend, daß das zu behandelnde Objekt zwischen einer Elektrode 1 und einer Gegenelektrode 7 angeordnet ist und sich somit mitten im erzeugten Plasma findet.

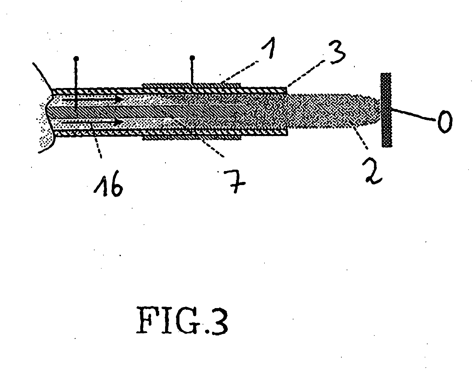

In Figur 3 (Stand der Technik beispielsweise gemäß EP 1 800 711 A1) ist zu erkennen, dass über eine röhrenförmige Zuführung eines zu ionisierenden Gasen mittels Elektrode 1 und Gegenelektrode 7 über eine Gasentladung ein entsprechender Plasmastrahl 2 entsteht, der direkt auf ein zu behandeindes Objekt geleitet wird.

Bei den in den Figuren 1 bis 2 dargestellten Prinzipien befindet sich grundsätzlich zwischen Elektrode und dem zu behandelnden Objekt ein entsprechendes Festkörperdielektrikum, wobei in Figur 2 darüber hinaus auch zwischen Gegenelektrode 7 und dem zu behandelnden Objekt ein entsprechendes Festkörperdielektrikum 3 vorhanden ist.

Die folgenden Figuren erläutern beispielhaft verschiedene erfindungsgemäße Ausführungsformen.

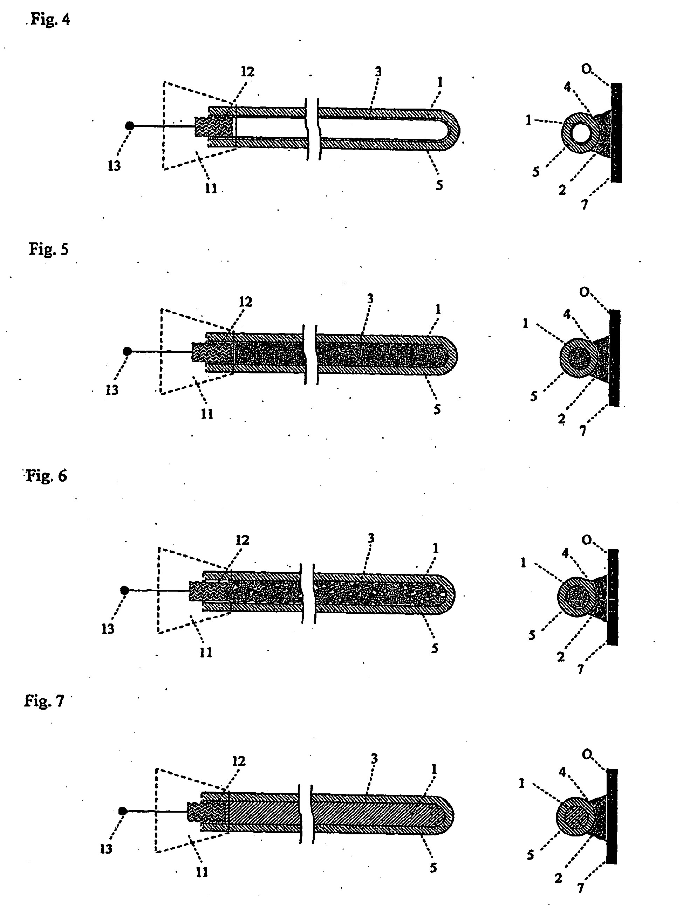

In Figur 4 besteht das dielektrische Material aus Glas, Keramik oder Kunststoff und ist als flexible Hohlfaser 5 ausgestaltet, wobei die Innenwandung der Hohlfaser 5 beschichtet ist mit einem elektrisch leitenden Material wie beispielsweise Metalle, dotierte Halbleiter oder leitende Metalloxidschichten (ITO) (Indium-Tin-oxid), wobei die Beschichtung als Elektrode 1 fungiert. Bei einer solchen Ausgestaltung wird in der Regel bei Anwendung lediglich einer Hohlfaser 5 das zu behandelnde Objekt als Gegenelektrode fungieren.

Die in Figur 5 aufgezeigte Ausführungsfom unterscheidet sich zu der in Figur 4 lediglich dahingehend, dass die Elektrode 1 als Vollmaterial ausgestaltet ist und aus leitenden Materialien wie Metalle und/oder Metall-Legierungen oder ähnliches besteht.

Die in Figur 6 aufgezeigte Ausführungsform unterscheidet sich zu denen in den Figuren 4 und 5 dargestellten Dahingehend, dass die Elektrode 1 als Pulver, bestehend aus leitenden Materialien wie Metalle und/oder Metall-Legierungen oder ähnliches, ausgestaltet ist.

Die in Figur 7 dargestellte Ausführungsform unterscheidet sich zu den vorherigen dadurch, daß die Elektrode als ionisiertes Gas, beispielsweise Edelgase oder andere Inertgase oder Gasgemische davon oder aus anderen ionisierbaren Gasen besteht, wobei das ionisierte Gas beispielsweise dadurch erzeugt wird, daß durch das Anlegen einer Hochspannung größer als die Durchbruchspannung das Gas ionisiert (Plasma) wird. Das ionisierte Gas ist nun elektrisch leitend und kann somit als Elektrode genutzt werden.

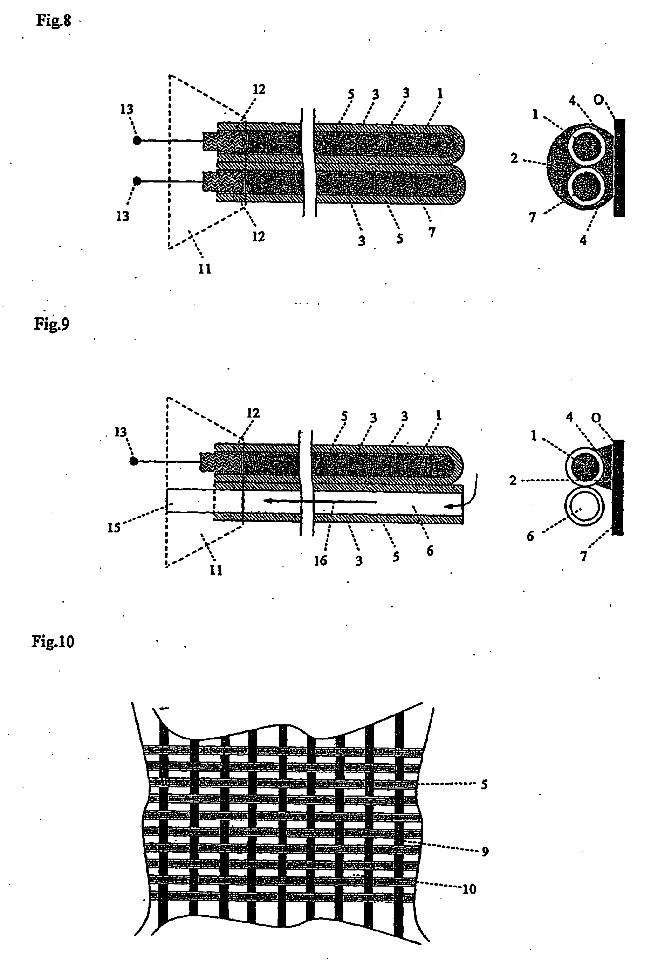

Die in Figur 8 aufgezeigte Ausführungsform unterscheidet sich von den in den Figuren 4, 5, 6 und 7 aufgezeigten dadurch, daß zwei entsprechende Hohlfasern 5 aus dielektrischem Material und jeweils mit vollmaterialigen Elektroden benachbart zueinander in Längsrichtung angeordnet sind, so daß bei Anlegen einer entsprechenden Spannung die obere Elektrode als Elektrode 1 und die untere Elektrode als Gegenelektrode 7 fungieren, so daß sich durch die geometrische Anordnung dieser beiden Hohlfasern eine bestimmte Geometrie des Plasmas einstellt, wobei darüber hinaus auch mehrere Hohlfasern denkbar sind, um eine entsprechende Geometrie des Plasmas zu erzeugen.

Figur 9 unterscheidet sich zu den in den Figuren 4, 5, 6 und 7 aufgezeigten Ausführungsformen dahingehend, daß in Längsrichtung benachbart zur Hohlfaser 5 eine entsprechende Absaugeinrichtung 6 angeordnet ist, so daß ggf. unerwünschte Komponenten, beispielsweise erzeugte Sauerstoffradikale, schnell von dem zu behandelnden Objekt entfernt werden, beispielsweise, um empfindliche Hautpartikel nicht zu reizen.

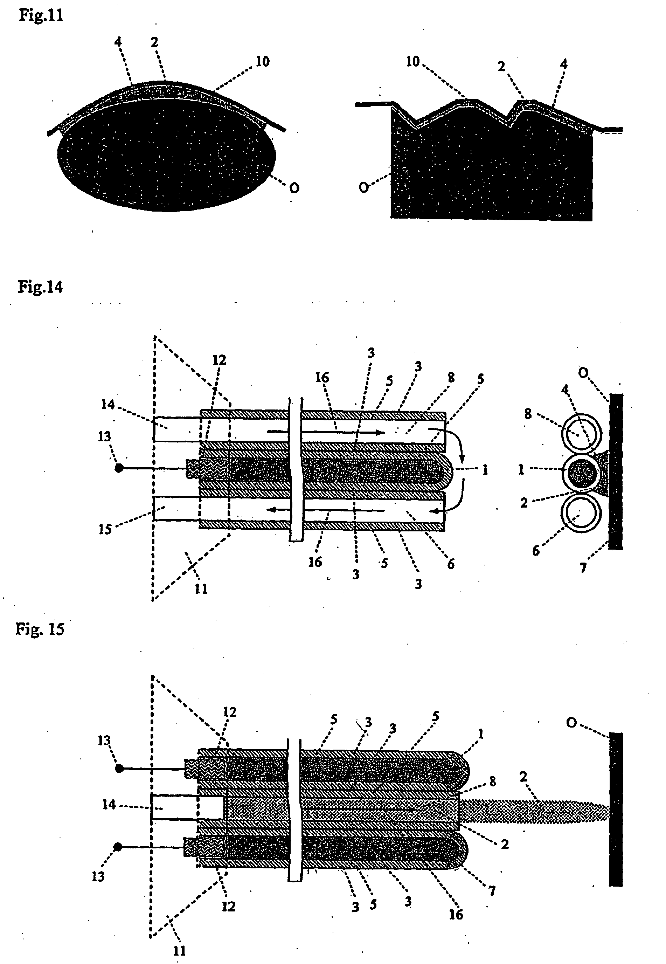

Figur 14 unterscheidet sich zu denen in den Figuren 4, 5, 6 und 7 aufgezeigten Ausführungsformen dahingehend, daß in Längsrichtung benachbart zur Hohlfaser 5 eine entsprechende, flexible Gasabsaugeinrichtung 6 und eine flexible Gaszuführungseinrichtung 8 angeordnet ist, so daß ggf. unerwünschte Komponenten, beispielsweise erzeugte Sauerstoffradikale, schnell von dem zu behandelnden Objekt entfernt werden aber auch um gezielt Gase zuzuführen, um beispielsweise das Behandlungsareal zu kühlen und/oder gezielt Reaktionen hervorzurufen.

In Figur 10 ist zu erkennen, daß mehrere Hohlfasern 5 aus dielektrischem Material oder mit einer dielektrischen Beschichtung aus Glas, Keramik oder Kunststoff und mit Elektroden versehen, beispielsweise in Form einer inneren Beschichtung (s. Ausführungsform der Figur 4) im Verbund mit weiteren Stützelementen 9 in Form von Fasern ein gewebeartiges Element 10 bilden, so daß auch bei schwierigen Topologien (s. Figur 11) eine entsprechend adäquate und angepaßte Formung und somit Applikation möglich ist.

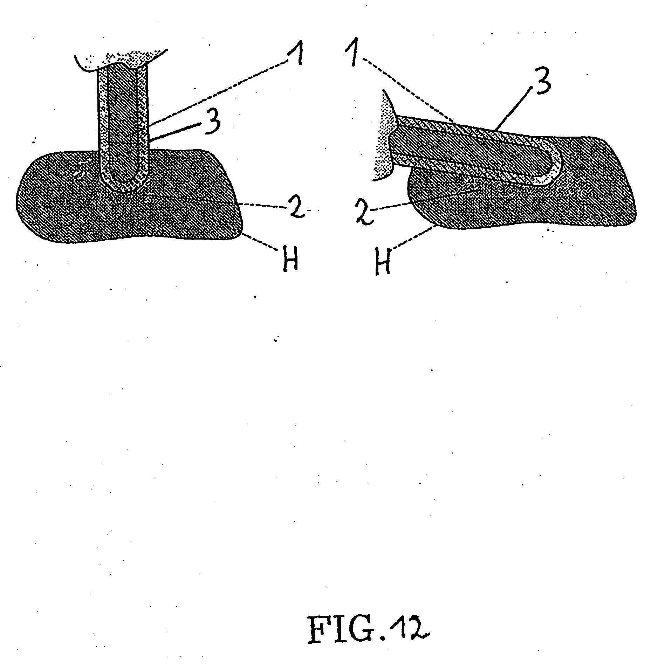

Schließlich ist in Figur 12 skizzenhaft eine übliche Applikation der erfindungsgemäßen Vorrichtung bezüglich eines Teils eines Hautareals H (in diesem Fall ist die Haut H das zu behandelnde Objekt O), als Gegenelektrode und Objekt fungierend, dargestellt.

In den Figuren 15 bis 17 sind unterschiedliche Beispiele, nicht im Bereich der Erfindung dargestellt, die sich zu den vorherigen dadurch unterscheiden, daß Elektrode/Elektroden und/oder Gaszuführungseinrichtung derart angeordnet sind, daß eine freie Plasmaflamme ausgebildet wird. Die aus der flexiblen Vorrichtung austretende freie Plasmaflamme des Plasmas 2 kann zur direkten topischen Applizierung verwendet werden.

Bei der in Figur 15 dargestellten Beispiel ist das Plasma in Bezug auf Elektrode 1 und Gegenelektrode 7 dielektrisch in direktem Kontakt gehindert durch entsprechende Festkörperdielektrika 3.

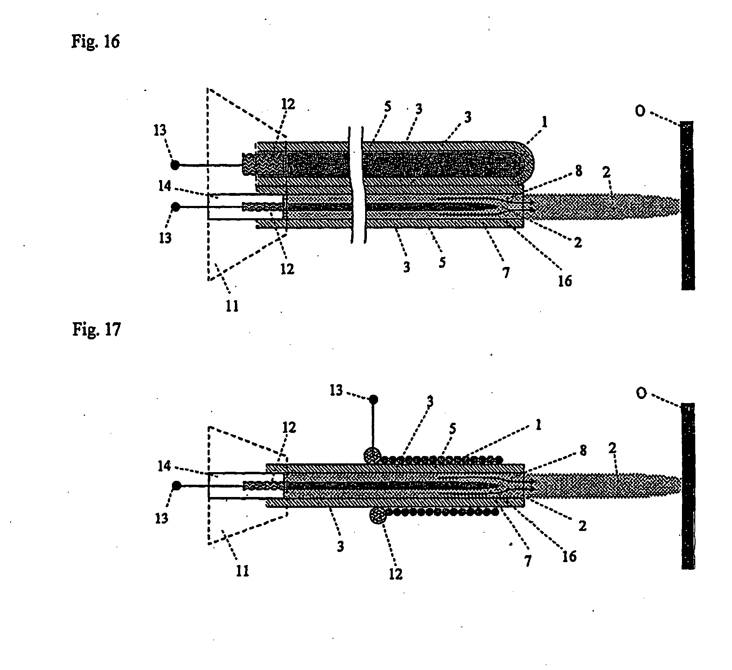

Bei den Beispiele in den Figuren 16 und ₁₇ ist lediglich eine einfache dielektrische Behinderung dahingehend gegeben, daß das Plasma an einem unmittelbaren direkten Kontakt mit der Elektrode 1 durch das Festkörperdielektrikum 3 gehindert wird, jedoch einen direkten Kontakt mit der Gegenelektrode 7 aufweist, da diese sich im Plasma selbst befindet und beispielsweise als elektrisch leitend flexibler Draht ausgestaltet ist.

Die Beispiel in Figur 17 unterscheidet sich im wesentlichen von der in Figur 16 dahingehend, daß die Elektrode 1 spiralförmig als Außenelektrode um das Festkörperdielektrikum (hier: Hohlfasermaterial) angeordnet ist, um eine gewisse mechanische Flexibilität zu erhalten bzw. zu unterstützen. Selbstverständlich ist es auch denkbar, daß die in den Figuren 15 bis 17 gezeigten Beispiele mit einer Gasabsaugungseinrichtung wie in Figur 14 gezeigt ausgestattet sein kann.

### Bezugszeichenliste

- O: - Objekt
- H: - Haut

- 1: - Elektrode
- 2: - Plasma
- 3: - Festkörperdielektrikum
- 4: - Wirkoberfläche
- 5: - Hohlfaser
- 6: - Gasabsaugeinrichtung
- 7: - Gegenelektrode
- 8: - Gaszuführungseinrichtung
- 9: - Stützelement
- 10: - Gewebeartiges Element
- 11: - Halterung
- 12: - Kontaktierung
- 13: - Anschluß, elektrischer
- 14: - Gaseinlaß
- 15: - Gasauslaß
- 16: - Gasfluß

## Patentansprüche

1. Vorrichtung zur Behandlung von Oberflächen von als Gegenelektrode fungierenden Objekten mit einem mittels einer Elektrode (1) über ein Feststoff-Dielektrikum (3) durch eine dielektrisch behinderte Gasentladung erzeugten Plasma (2) und mit einer Wirk-Oberfläche (4), die während der Behandlung unmittelbar an das Plasma (2) angrenzt, wobei das Dielektrikum (3) mit einer Hohlfaser (5) gebildet ist, deren Mantelwandung die Wirk-Oberfläche (4) aufweist und die Wirk-Oberfläche (4) reversibel formveränderbar ist, **dadurch gekennzeichnet, dass** die Mantelwandung einstückig an einem Ende in eine die Hohlfaser (5) verschließende Stirnwandung übergeht.

2. Vorrichtung nach Anspruch 1, **dadurch gekennzeichnet, dass** die Elektrode (1) zumindest teilweise direkt an dem Dielektrikum (3) anliegt.

3. Vorrichtung nach Anspruch 1 oder 2, **dadurch gekennzeichnet, dass** die Elektrode (1) zumindest teilweise mittels eines Spacers beabstandet zum Dielektrikum (3) ist.

4. Vorrichtung nach Anspruch 2, **dadurch gekennzeichnet, dass** die Elektrode (1) zumindest teilweise als Beschichtung am Dielektrikum (3) anliegt.

5. Vorrichtung nach einem der Ansprüche 1 bis 4, **dadurch gekennzeichnet, dass** die Elektrode (1) mit einem leitenden Vollmaterial am Dielektrikum (3) anliegt.

6. Vorrichtung nach einem der Ansprüche 1 bis 4, **dadurch gekennzeichnet, dass** die Elektrode (1) mit einem leitenden Granulat und/oder Pulver an dem Dielektrikum (3) anliegt.

7. Vorrichtung nach einem der Ansprüche 1 bis 4, **dadurch gekennzeichnet, dass** die Elektrode (1) mit einem elektrisch leitenden Fluid an die Dielektrikum (3) anliegt.

8. Vorrichtung nach einem der Ansprüche 1 bis 7, **dadurch gekennzeichnet, dass** die Hohlfaser (5) eine flexible Hohlfaser ist.

9. Vorrichtung nach einem der Ansprüche 1 bis 8, **gekennzeichnet durch** eine Gasabsaugeinrichtung (6) und/oder eine Gaszuführungseinrichtung (8).

10. Vorrichtung nach Anspruch 9, **dadurch gekennzeichnet, dass** die Gasabsaugeinrichtung (6) und/oder die Gaszuführungseinrichtung (8) flexibel ausgestattet ist.

11. Vorrichtung nach einem der Ansprüche 1 bis 10, **dadurch gekennzeichnet, dass** das Dielektrikum (3) im Verbund mit weiteren Stützelementen (9) ein aus Fasern gebildetes flexibles gewebeartiges Element (10) ist, das großflächig auf die zu behandelnde Oberfläche auflegbar ist.

## Claims

1. Apparatus for treatment of surfaces of objects functioning as counterelectrode with a plasma (2) which is produced by means of an electrode (1) over a solid dielectric (3) by a dielectric-barrier gas discharge, and having an active surface (4) which is directly adjacent to the plasma (2) during the treatment, wherein the dielectric (3) is formed with a hollow fibre (5), the lateral wall of which has the active surface (4), and the active surface (4) has a reversibly variable shape, **characterized in that** the lateral wall undergoes transition integrally at one end into an end wall that closes the hollow fibre (5).

2. Apparatus according to Claim 1, **characterized in that** the electrode (1) rests at least partially directly on the dielectric (3).

3. Apparatus according to one of Claims 1 and 2, **characterized in that** the electrode (1) is separated at least partially by means of a spacer from the dielectric (3).

4. Apparatus according to Claim 2, **characterized in that** the electrode (1) rests at least partially on the dielectric (3), as a coating.

5. Apparatus according to one of Claims 1 to 4, **characterized in that** the electrode (1) rests on the dielectric (3) with a conductive solid material.

6. Apparatus according to one of Claims 1 to 4, **characterized in that** the electrode (1) rests on the dielectric (3) with a conductive granulate and/or powder.

7. Apparatus according to one of Claims 1 to 4, **characterized in that** the electrode (1) rests on the dielectric (3) with an electrically conductive fluid.

8. Apparatus according to one of Claims 1 to 7, **characterized in that** the hollow fibre (5) is a flexible hollow fibre.

9. Apparatus according to one of Claims 1 to 8, **characterized by** a gas extraction device (6) and/or a gas supply device (8).

10. Apparatus according to Claim 9, **characterized in that** the gas extraction device (6) and/or the gas supply device (8) are/is flexible.

11. Apparatus according to one of Claims 1 to 10, **characterized in that** the dielectric (3) in association with further supporting elements (9) is a flexible fabric-like element (10) which is formed from fibres and which can be placed over a large area onto the surface to be treated.

## Revendications

1. Dispositif pour le traitement de surfaces d'objets faisant office de contre-électrode avec un plasma (2) engendré au moyen d'une électrode (1) via une décharge gazeuse entravée par le biais d'un diélectrique solide (3) et présentant une surface active (4) qui, pendant le traitement, est immédiatement adjacente au plasma (2), dans lequel le diélectrique (3) est réalisé avec une fibre creuse (5) dont la paroi enveloppe présente la surface active (4) et la surface active (4) peut être modifiée de façon réversible quant à sa forme, **caractérisé en ce que** la paroi enveloppe se transforme d'un seul tenant à une extrémité pour donner la paroi frontale qui obture la fibre creuse (5).

2. Dispositif selon la revendication 1, **caractérisé en ce que** l'électrode (1) est appliquée au moins partiellement directement sur le diélectrique (3).

3. Dispositif selon la revendication 1 ou 2, **caractérisé en ce que** l'électrode (1) est au moins partiellement écartée du diélectrique (3) au moyen d'un élément d'écartement.

4. Dispositif selon la revendication 2, **caractérisé en ce que** l'électrode (1) est appliquée sur le diélectrique (3) au moins partiellement sous forme de revêtement.

5. Dispositif selon l'une des revendications 1 à 4, **caractérisé en ce que** l'électrode (1) est appliquée sur le diélectrique (3) avec un matériau plein conducteur.

6. Dispositif selon l'une des revendications 1 à 4, **caractérisé en ce que** l'électrode (1) est appliquée sur le diélectrique (3) avec un granulé conducteur et/ou une poudre conductrice.

7. Dispositif selon l'une des revendications 1 à 4, **caractérisé en ce que** l'électrode (1) est appliquée sur le diélectrique (3) avec un fluide électriquement conducteur.

8. Dispositif selon l'une des revendications 1 à 7, **caractérisé en ce que** la fibre creuse (5) est une fibre creuse flexible.

9. Dispositif selon l'une des revendications 1 à 8, **caractérisé par** un dispositif d'aspiration de gaz (6) et/ou par un dispositif d'amenée de gaz (8).

10. Dispositif selon la revendication 9, **caractérisé en ce que** le dispositif d'aspiration de gaz (6) et/ou le dispositif d'amenée de gaz (8) est conçu de manière flexible.

11. Dispositif selon l'une des revendications 1 à 10, **caractérisé en ce que** le diélectrique (3) est, en association avec d'autres éléments de soutien (9), un élément flexible semblable à un textile (10) formé à partir de fibres, qui peut être appliqué en grande surface sur la surface à traiter.
